# FASCICULE DE BREVET EUROPEEN

(11) **EP 2 544 633 B1**
(45) Date de publication et mention de la délivrance du brevet: **19.11.2014**
(21) Numéro de dépôt: 11712977.5
(22) Date de dépôt: 08.03.2011
(51) Int. Cl.: A61F 2/42

(54) **IMPLANT D'ARTHRODESE**
ARTHRODESEIMPLANTAT
ARTHRODESIS IMPLANT

(30) Priorité: 09.03.2010 FR 1051673
(43) Date de publication de la demande: 16.01.2013
(73) Titulaire: Synchro Medical, 68920 Wettolsheim Les Erlen (FR)
(72) Inventeur: AVEROUS, Christophe, F-67206 Mittelhausbergen (FR); CERMOLACCE, Christophe, F-13008 Marseille (FR); DETERME, Patrice, F-31400 Toulouse (FR); DIEBOLD, Patrice, F-54000 Nancy (FR); GUILLO, Stéphane, F-33000 Bordeaux (FR); ROCHER, Hubert, F-33600 Pessac (FR); ROY, Christophe, F-26300 Chatuzange le Goubet (FR)
(74) Mandataire: Verriest, Philippe
(86) Numéro de dépôt international: PCT/FR2011/050473
(87) Numéro de publication internationale: WO 2011/110784

(56) Documents cités:
- WO-A1-2006/109004
- DE-A1- 1 805 665
- FR-A1- 2 913 876
- US-B1- 7 041 106

## Description

La présente invention porte sur un implant médical destiné à relier deux os entre eux. En particulier, l'invention porte sur un implant articulaire orthopédique du pied ou de la main, destiné à relier et fixer entre elles deux phalanges adjacentes.

Certaines pathologies, comme l'Hallux Valgus ou les métatarsalgies (ou orteils en griffes), induisent au niveau du pied une déformation des orteils : en particulier, il arrive que deux phalanges adjacentes d'un orteil ne conservent pas leur alignement naturel et forment entre elles un angle déformant l'orteil de façon constante. Une telle déformation peut avoir lieu dans un plan vertical, et le patient souffre alors de métatarsalgie : l'orteil adopte une configuration « en griffe » dans laquelle il est replié sur lui-même en permanence. La déformation peut alternativement avoir lieu dans un plan horizontal : le patient souffre alors d'Hallux Valgus : l'orteil, en particulier le gros orteil, forme une proéminence à l'extérieur du pied.

Ces pathologies provoquent des douleurs de l'avant-pied, des cors, des gênes fonctionnelles : le patient a du mal à marcher correctement : il peut également avoir des difficultés pour se chausser.

Pour corriger ces pathologies, il est d'usage de pratiquer une arthrodèse interphalangienne : cette technique consiste à bloquer par voie chirurgicale l'articulation formée par les deux phalanges ayant perdu leur alignement naturel, en les réalignant l'une sur l'autre et en les soudant en une seule par fusion osseuse, les deux phalanges étant maintenues accolées l'une à l'autre durant le temps nécessaire à la fusion osseuse au moyen d'un implant.

L'implant utilisé dans ce type de correction présente donc deux parties, une partie destinée à être introduite dans la première phalange, et une deuxième partie destinée à être introduite dans la deuxième phalange : les deux phalanges sont ainsi accolées l'une à l'autre. L'implant doit permettre la fusion des deux os, ou phalanges, ainsi accolés.

Des implants destinés à réaliser des arthrodèses interphalangiennes ont déjà été décrits. Ainsi, le document WO2008/129214 décrit un implant comprenant deux zones d'ancrage osseux opposées, chaque zone comprenant deux branches parallèles, l'implant présentant une forme globale en H.

Toutefois, les implants existant n'assurent pas toujours la stabilité nécessaire dans les trois directions de l'espace, de l'ensemble première phalange, deuxième phalange et implant, pendant le temps nécessaire, après implantation, jusqu'à la fusion des deux phalanges entre elles. Or, si l'implant ou une des deux phalanges est déplacé pendant ce laps de temps, la fusion des deux phalanges se fera de façon incorrecte et le traitement échouera.

Il importe donc que l'implant, une fois implanté, assure une bonne stabilité tridimensionnelle et un bon maintien de l'ensemble première phalange, deuxième phalange et implant, au moins jusqu'à ce que la fusion entre elles des deux phalanges dans la position voulue par le chirurgien ait pu avoir lieu.

La présente invention propose un implant présentant une structure apte à conférer à l'implant à la fois une flexibilité et une rigidité lui permettant d'assurer, une fois implanté, une stabilité tridimensionnelle à l'ensemble première phalange, deuxième phalange et implant, au moins durant le temps nécessaire à la fusion osseuse entre les deux phalanges, c'est-à-dire pendant une durée d'environ six semaines. L'implant selon l'invention permet ainsi de restituer à l'organe traité son anatomie naturelle, en particulier l'angle naturel entre les deux os ou phalanges ainsi ressoudés.

La présente invention porte sur un implant médical destiné à permettre la fusion osseuse entre un premier os et un deuxième os, ledit implant comprenant une première partie, de forme globalement allongée et présentant un axe longitudinal A, destinée à être introduite dans le premier os et comprenant des premiers moyens de fixation dudit implant dans ledit premier os, et une deuxième partie, de forme également globalement allongée et présentant un axe longitudinal B, destinée à être introduite dans le deuxième os et comprenant des deuxièmes moyens de fixation dudit implant dans ledit deuxième os, lesdites première et deuxième parties étant reliées entre elles par un noyau central, caractérisé en ce que ledit noyau central est un solide plein dont la section en coupe par un plan perpendiculaire audit axe longitudinal A présente sensiblement une forme d'étoile à au moins trois branches, ladite première partie présentant au moins trois pattes, chaque patte s'étendant sensiblement selon ledit axe longitudinal A à partir de l'extrémité libre d'une des trois branches dudit noyau central.

Du fait de sa structure, l'implant selon l'invention assure, une fois implanté, une stabilité optimale de l'ensemble premier os, deuxième os et implant, durant tout le temps nécessaire à la fusion entre les deux os. L'implant selon l'invention est particulièrement adapté à la réalisation d'une arthrodèse entre deux phalanges du pied ou de la main. La première partie, de forme allongée, de l'implant selon l'invention est destinée à être introduite par exemple dans le canal médullaire d'une première phalange, et la deuxième partie, également de forme allongée, de l'implant selon l'invention, est destinée à être introduite dans le canal médullaire de la deuxième phalange, adjacente à la première. Grâce à sa structure spécifique, en particulier grâce à la présence de trois pattes dans la première partie de l'implant, chacune s'étendant à partir d'une branche du noyau central plein, l'implant selon l'invention assure une stabilité parfaite dans les trois directions de l'espace, de l'ensemble première phalange, deuxième phalange et implant, et ce dans la position voulue par le chirurgien au moment de l'implantation. Par ailleurs, le noyau central en solide plein de l'implant selon l'invention assure à la fois une rigidité et une flexibilité de l'implant, permettant une mise en place facilitée de l'implant et un maintien fiable de l'ensemble premier os, deuxième os et implant dans une position déterminée.

Selon la présente demande, on entend par « forme d'étoile à au moins trois branches », une forme ayant un point de jonction à partir duquel s'étendent, sensiblement radialement, au moins trois branches, lesdites branches étant séparées les unes des autres par des angles réguliers ou non. Par exemple, dans une forme de réalisation, ladite section en coupe dudit noyau central présente une forme en T : dans un tel cas, les trois branches du T sont espacées entre elles d'angles différents, à savoir deux angles de 90° et un angle de 180°. Dans une autre forme de réalisation, ladite section en coupe dudit noyau central présente une forme en Y. Dans d'autres formes de réalisation, la section en coupe du noyau central présente une forme d'étoiles à plus de trois branches, par exemple à quatre ou cinq branches.

Le noyau central de l'implant selon l'invention est un solide plein : ainsi, le point de jonction de l'étoile est constitué de matériau. Dans une forme de réalisation, ledit implant est monobloc. L'implant selon l'invention peut être formé d'un matériau choisi parmi les polyéthercétones (PEEK), le titane, l'acier inoxydable, les acides polylactiques, et leurs mélanges. Par exemple, l'implant selon l'invention peut être biorésorbable ou non.

L'implant selon l'invention peut par exemple être réalisé par moulage injection, ou encore par usinage.

Dans une forme de réalisation, lesdites pattes présentant globalement la forme d'un parallélépipède allongé, au moins une patte présente une section transversale réduite au niveau de sa jonction avec le noyau central. De préférence, les trois pattes présentent chacune une section transversale réduite au niveau de sa jonction avec le noyau central. Une telle forme de réalisation permet de conférer à l'implant une élasticité et une flexibilité permettant de le mettre en place aisément. Par ailleurs, du fait de la structure solide du noyau central, l'implant conserve une rigidité lui permettant d'assurer une bonne stabilité de l'ensemble premier os, deuxième os et implant sur le site d'implantation.

Dans une forme de réalisation, ladite deuxième partie présente au moins deux jambes s'étendant selon l'axe longitudinal B à partir dudit noyau central. Alternativement, ladite deuxième partie présente trois jambes s'étendant selon l'axe longitudinal B à partir dudit noyau central.

Dans une forme de réalisation, la section en coupe dudit noyau central par un plan perpendiculaire audit axe longitudinal B présentant sensiblement ladite forme d'étoile à au moins trois branches, chaque dite jambe s'étend sensiblement selon ledit axe longitudinal B à partir de l'extrémité libre d'une des branches dudit noyau central.

Une telle forme de réalisation permet de renforcer la stabilité dans les trois directions de l'espace de l'ensemble premier os, deuxième os et implant, dans la position voulue par le chirurgien, une fois que l'implant selon l'invention est implanté et que les deux os à souder sont accolés l'un à l'autre.

Dans une forme de réalisation, lesdites jambes présentant globalement la forme d'un parallélépipède allongé, chaque jambe présente une section transversale réduite au niveau de sa jonction avec le noyau central. Comme vu précédemment, une telle forme de réalisation permet de conférer à l'implant une élasticité et une flexibilité permettant de le mettre en place aisément. Par ailleurs, du fait de la structure solide du noyau central, l'implant conserve une rigidité lui permettant d'assurer une bonne stabilité de l'ensemble premier os, deuxième os et implant sur le site d'implantation.

Dans une forme de réalisation, lesdits premiers moyens de fixation comprennent des crans d'accroche situés sur lesdites pattes. Par exemple, ces crans d'accroche sont situés sur les faces externes des parallélépipèdes formant ces pattes, afin que ces dernières se fixent dans l'os dans lequel elles sont introduites. De même, les deuxièmes moyens de fixation peuvent comprendre des crans d'accroche situés sur lesdites jambes, par exemples sur les faces externes des parallélépipèdes formant lesdites jambes afin que ces dernières se fixent dans l'os dans lequel elles sont introduites. Les crans d'accroche présentent de préférence des arêtes dirigées vers le noyau central afin de compresser chaque os en direction de l'autre os, contre lequel il est accolé, lorsque l'implant selon l'invention est implanté.

Les avantages de la présente invention vont ressortir plus clairement de la description qui suit et des dessins annexés dans lesquels :
- La Figure 1 est une vue en perspective d'une forme de réalisation de l'implant selon l'invention,
- La figure 2 est une vue de côté de l'implant de la figure 1,
- La figure 3 est une vue en coupe selon le plan II' de la figure 2 et en perspective de la première partie de l'implant de la figure 1,
- La figure 4 est une vue en perspective de l'arrière de la première partie de l'implant montrée sur la figure 3,
- La figure 5 est une vue de dessous de l'implant de la figure 1,
- La figure 6 est une vue en perspective d'une autre forme de réalisation de l'implant selon l'invention,
- La figure 7 est une vue schématique de l'implant de la figure 1 une fois implanté entre deux os.

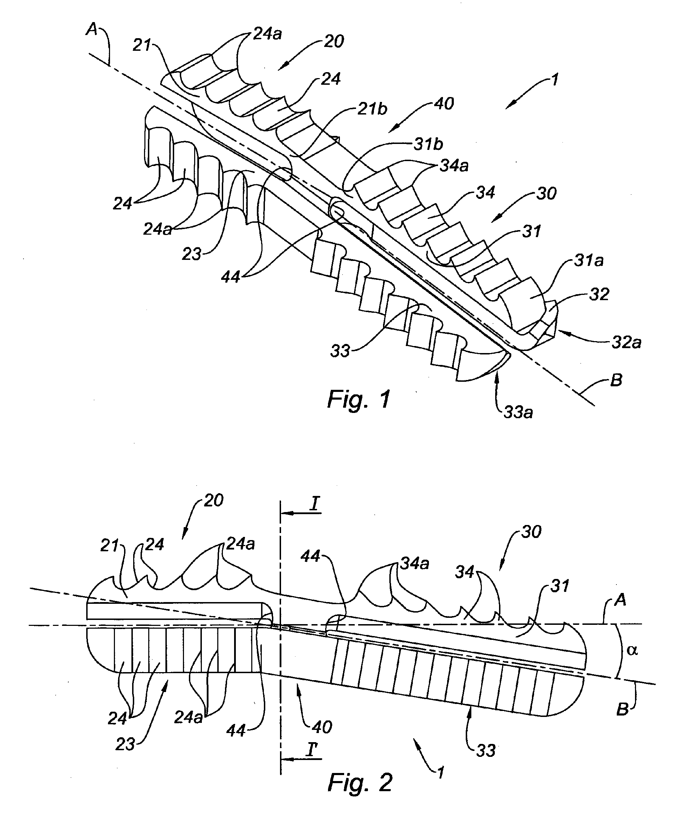

En référence aux figures 1 à 3 est représenté un implant 1 selon l'invention pour la réalisation d'une arthrodèse. L'implant 1 comprend une première partie 20, de forme globalement allongée, présentant un axe longitudinal A, une deuxième partie 30, également de forme globalement allongée et présentant un axe longitudinal B, et un noyau central 40 reliant la première partie 20 à la deuxième partie 30. Sur l'exemple représenté, l'axe longitudinal A et l'axe longitudinal B forment entre eux un angle α : cet angle α peut varier d'environ 0 à 30°, par exemple de 10 à 20°, en particulier pour ajuster l'implant de l'invention à l'anatomie de la partie du corps humain à traiter, par exemple le pied ou la main. Dans une forme de réalisation non représentée, l'angle α peut être nul : dans un tel cas, la première partie et la deuxième partie sont situées dans l'alignement l'une de l'autre.

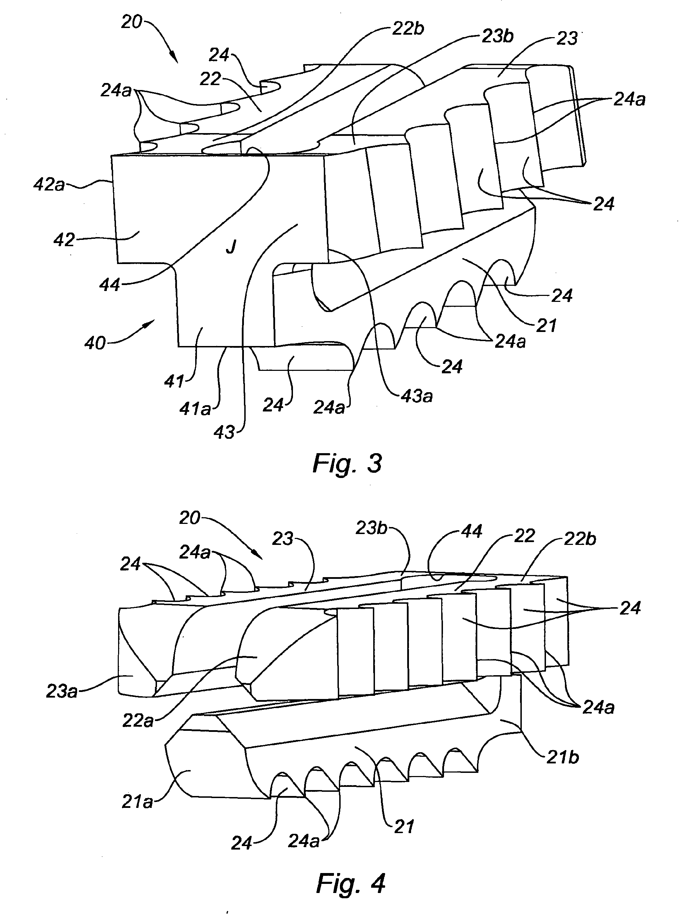

Comme il apparaît de la figure 3, le noyau central 40 est un solide plein dont la section en coupe par le plan II' (voir figure 2) perpendiculaire à l'axe longitudinal A présente sensiblement une forme d'étoile à au moins trois branches 41, 42 et 43 : sur l'exemple représenté, cette étoile a la forme d'un T, la branche 41 correspondant à la barre verticale du T, chaque branche 42 et 43 correspondant chacune à une extrémité de la barre transversale du T. Ainsi, chaque branche 41, 42 et 43 s'étend approximativement radialement à partir d'un point de jonction représenté par la lettre J sur la figure 3, jusqu'à leur extrémité libre respective (41 a, 42a, 43a).

Dans une forme de réalisation non représentée, l'étoile pourrait avoir globalement la forme d'un Y. Alternativement, l'étoile pourrait comprendre plus de trois branches. Les branches de l'étoile s'étendent sensiblement radialement à partir de leur point de jonction J, et sont espacées les unes par rapport aux autres par des angles réguliers ou non. Dans la forme de réalisation représentée sur la figure 3, la branche 41 est espacée de chaque branche 42 et 43 par un angle de 90° et les deux branches 42 et 43 sont espacées par un angle de 180°.

Comme il apparaît des figures 3 et 4, la première partie, dans l'exemple représenté, comprend trois pattes (21, 22, 23) s'étendant chacune respectivement de l'extrémité libre (41 a, 42a, 43a) des trois branches (41, 42, 43) selon l'axe longitudinal A (voir figure 1).

Les pattes (21, 22, 23) présentent toutes la forme globale d'un parallélépipède allongé, dont l'extrémité libre (21a, 22a, 23a) présente une forme en portion de cône. Une telle forme des extrémités libres (21 a, 22a, 23a) des pattes (21, 22, 23) permet une insertion facilitée dans l'os dans lequel la première partie 20 est destinée à être introduite. La section du parallélépipède, en dehors de la région de son extrémité libre, peut par exemple être globalement carrée, rectangulaire, triangulaire, trapézoïdale. Dans une forme de réalisation non représentée, les pattes pourraient avoir la forme globale d'un cylindre ayant une section de forme ovalaire ou encore ronde. Sur l'exemple représenté, les trois pattes (21, 22, 23) s'étendent parallèlement les unes aux autres. Dans une forme de réalisation non représentée, les pattes (21, 22, 23) pourraient s'étendre selon des axes respectifs s'écartant légèrement de l'axe longitudinal A.

Sur l'exemple représenté, chaque patte (21, 22, 23) est munie de crans d'accroche 24 : ces crans d'accroche constituent des premiers moyens de fixation destinés à assurer le maintien de la première partie 20 de l'implant 1 dans l'os dans lequel cette première partie est destinée à être introduite. Dans cette perspective, comme montré sur la figure 2, ces crans d'accroche 24 présentent chacun une arête 24a dirigée vers le noyau central 40 : comme il apparaîtra plus tard dans la description ci-dessous, la direction des arêtes 24a vers le noyau central 40 aide à accoler l'os dans lequel est introduite la première partie à l'os dans lequel la deuxième partie 30 de l'implant 1 est implantée.

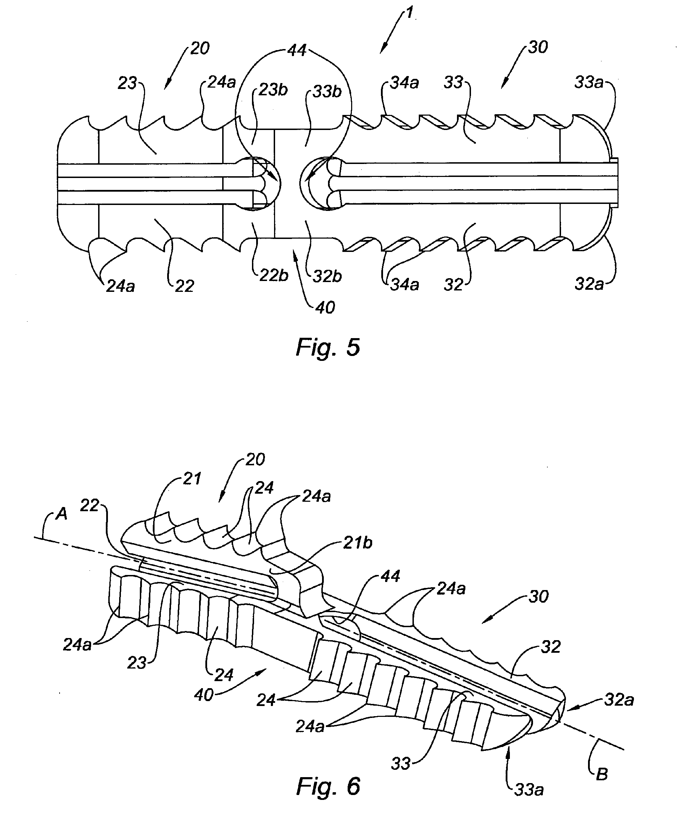

En référence aux figures 3 et 5, les trois pattes (21, 22, 23) présentent chacune une section transversale réduite (21 b, 22b, 23b) au niveau de sa jonction avec le noyau central 40. Ces sections réduites (21 b, 22b, 23b) forment en particulier des découpes curvilignes 44 au niveau du noyau central 40. De telles sections réduites des pattes, et notamment les découpes curvilignes 44 du noyau central 40 en résultant, permettent de conférer à l'implant 1 une élasticité et une flexibilité, en particulier autour du noyau central 40, permettant de mettre l'implant 1 en place aisément.

En référence aux figures 1 et 2, la deuxième partie 30, dans l'exemple représenté, comprend trois jambes (31, 32, 33) s'étendant chacune respectivement de l'extrémité libre (41 a, 42a, 43a) des trois branches (41, 42, 43) du noyau central 40 selon l'axe longitudinal B (voir figure 1). Ainsi, les jambes (31, 32,33) s'étendent globalement dans l'alignement, toutefois selon l'axe longitudinal B, des trois pattes (21, 22, 23) de la première partie 20.

Les jambes (31, 32, 33) présentent toutes la forme globale d'un parallélépipède allongé, dont l'extrémité libre (31 a, 32a, 33a) présente une forme en portion de cône. Comme vu précédemment pour la première partie 20, une telle forme des extrémités libres (31 a, 32a, 33a) des jambes (31, 32, 33) permet une insertion facilitée dans l'os dans lequel la deuxième partie 30 est destinée à être introduite. La section du parallélépipède, en dehors de la région de son extrémité libre, peut par exemple être globalement carrée, rectangulaire, triangulaire, trapézoïdale. Dans une forme de réalisation non représentée, les jambes pourraient avoir la forme globale d'un cylindre ayant une section de forme ovalaire ou encore ronde. Sur l'exemple représenté, les trois jambes (31, 32, 33) s'étendent parallèlement les unes aux autres. Dans une forme de réalisation non représentée, les jambes (31, 32, 33) pourraient s'étendre selon des axes respectifs s'écartant légèrement de l'axe longitudinal B.

Sur l'exemple représenté, chaque jambe (31, 32, 33) est munie de crans d'accroche 34 : ces crans d'accroche constituent des deuxièmes moyens de fixation destinés à assurer le maintien de la deuxième partie 30 de l'implant 1 dans l'os dans lequel cette deuxième partie 30 est destinée à être introduite. Dans cette perspective, comme montré sur la figure 2, ces crans d'accroche 34 présentent chacun une arête 34a dirigée vers le noyau central 40 : comme il apparaîtra plus tard dans la description ci-dessous, la direction des arêtes 34a vers le noyau central 40 permet de compresser l'os dans lequel est introduite la deuxième partie 30 contre l'os dans lequel la première partie 20 de l'implant 1 est implantée.

En référence aux figures 1 et 5, les trois jambes (31, 32, 33) présentent chacune une section transversale réduite (31 b, 32b, 33b) au niveau de sa jonction avec le noyau central 40. Ces sections réduites (31 b, 32b, 33b) forment en particulier des découpes curvilignes 44 au niveau du noyau central 40. Comme vu précédemment pour la première partie 20, de telles sections réduites des jambes (31, 32, 33), et notamment les découpes curvilignes 44 du noyau central 40 en résultant, permettent de conférer à l'implant 1 une élasticité et une flexibilité, en particulier autour du noyau central 40, permettant de mettre l'implant 1 en place aisément.

Dans l'exemple représenté, la deuxième partie 30 présente une longueur à peu près égale au double de la longueur de la première partie 20. Ainsi, l'implant 1 représenté sur les figures 1 à 5 est particulièrement adapté pour une arthrodèse interphalangienne, impliquant deux os (une première phalange et une deuxième phalange) de longueurs différentes. Dans une forme de réalisation non représentée, la première partie et la deuxième partie présentent des longueurs sensiblement égales.

En référence à la figure 6 est représentée une variante de réalisation de l'implant 1 selon l'invention, dans lequel la jambe de la deuxième partie 30 s'étendant à partir de l'extrémité libre 41 a de la barre verticale du T du noyau central 40 a été supprimée. La deuxième partie 30 comprend ainsi deux jambes (32, 33), s'étendant selon l'axe longitudinal B.

Les implants 1 décrits aux figures 1 à 6 sont de préférence monobloc. Ils peuvent être réalisés par exemple par injection moulage ou encore par usinage. Le matériau convenant pour la réalisation des implants 1 de ces figures peut être tout matériau biocompatible, métallique ou non. Par exemple, ce matériau peut être choisi parmi les polyéthercétones (PEEK), le titane, l'acier inoxydable, les acides polylactiques, et leurs mélanges. Grâce à leur structure spécifique, les implants selon l'invention garantissent une stabilité de l'ensemble premier os, deuxième os et implant, dans les trois directions de l'espace, dans la position voulue par le chirurgien, tout en présentant une bonne élasticité et une bonne flexibilité les rendant faciles à manipuler et à mettre en place.

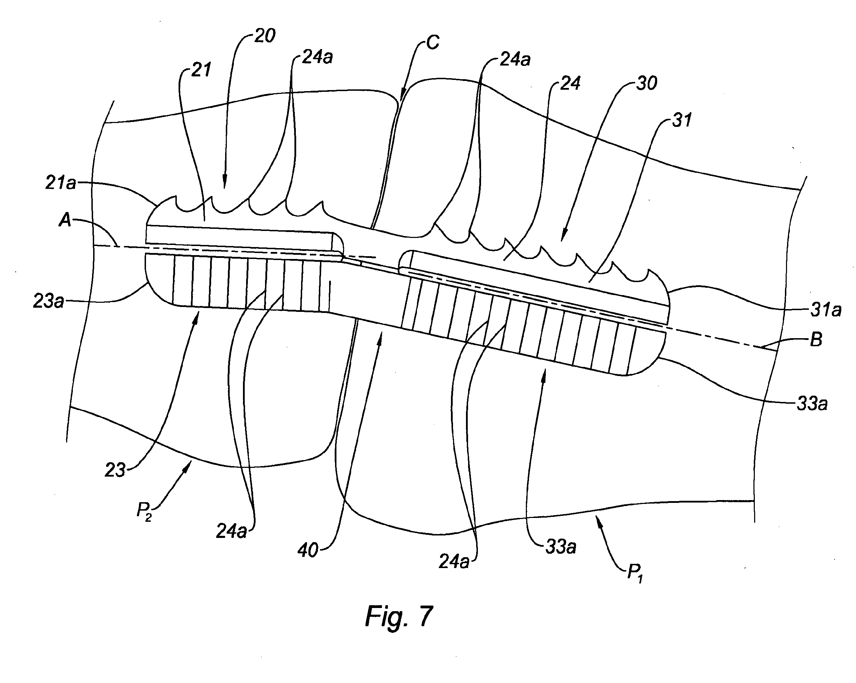

Sur la figure 7 est représenté l'implant 1 des figures 1 à 5 une fois implanté, dans le cas d'une arthrodèse concernant une articulation entre deux phalanges, la phalange P1 et la phalange P2. L'implant de la figure 6 pourrait être utilisé pour réaliser une arthrodèse de la même façon.

Comme il apparaît de la figure 7, la première partie 20 de l'implant 1 a été introduite dans le canal médullaire de la phalange P2 et la deuxième partie 30 de l'implant 1 a été introduite dans le canal médullaire de la phalange P1. Dans l'exemple représenté, la deuxième partie 30 étant plus longue que la première partie 20, on a de préférence introduit la deuxième partie 30 dans la phalange P1 préalablement à l'introduction de la première partie 20 dans la phalange P2. Dans une autre forme de réalisation non représentée, dans laquelle la première partie et la deuxième partie sont de longueur sensiblement égales et pour laquelle par exemple l'angle α (voir figure 2) est égal à 0°, l'ordre dans lequel les deux parties sont respectivement introduites dans les deux phalanges à souder peut être indifférent.

En référence à la figure 7, les crans d'accroche respectifs (24, 34) de la première partie 20 et de la deuxième partie 30, grâce à l'orientation respective de leurs arêtes (24a, 34a) poussent le premier os (la phalange P2) contre le deuxième os (la phalange P1) qui, comme on le voit sur la figure, sont accolés l'un à l'autre. Grâce à sa structure spécifique, l'implant 1 selon l'invention maintient l'ensemble phalange P1, phalange P2 et implant 1 dans la position ainsi déterminée par le chirurgien, au moins durant le temps nécessaire à la fusion osseuse entre la phalange P1 et la phalange P2, au niveau de leur surface initiale de contact, représenté par la lettre C sur la figure.

Il est ainsi possible, grâce à l'implant selon l'invention, de souder les deux phalanges P1 et P2 en une seule et de bloquer l'articulation entre ces deux phalanges et de traiter des pathologies telles que métatarsalgies ou encore Hallux Valgus.

## Revendications

1. Implant médical (1) destiné à permettre la fusion osseuse entre un premier os (P2) et un deuxième os (P1), ledit implant comprenant une première partie (20), de forme globalement allongée et présentant un axe longitudinal A, destinée à être introduite dans le premier os et comprenant des premiers moyens de fixation (24) dudit implant dans ledit premier os, et une deuxième partie (30), de forme également globalement allongée et présentant un axe longitudinal B, destinée à être introduite dans le deuxième os et comprenant des deuxièmes moyens de fixation (34) dudit implant dans ledit deuxième os, lesdites première et deuxième parties (20, 30) étant reliées entre elles par un noyau central (40), **caractérisé en ce que** ledit noyau central est un solide plein dont la section en coupe par un plan perpendiculaire audit axe longitudinal A présente sensiblement une forme d'étoile à au moins trois branches (41, 42, 43), ladite première partie présentant au moins trois pattes (21, 22, 23), chaque patte s'étendant sensiblement selon ledit axe longitudinal A à partir de l'extrémité libre (41 a, 42a, 43a) d'une des branches dudit noyau central.

2. Implant (1) selon la revendication 1, **caractérisé en ce que** ladite section en coupe dudit noyau central présente une forme en T.

3. Implant (1) selon la revendication 1, **caractérisé en ce que** ladite section en coupe dudit noyau central présente une forme en Y.

4. Implant (1) selon l'une quelconque des revendications précédentes, **caractérisé en ce que** ledit implant est monobloc.

5. Implant (1) selon l'une quelconque des revendications précédentes, **caractérisé en ce que** lesdites pattes présentant globalement la forme d'un parallélépipède allongé, au moins une patte présente une section transversale réduite (21 a, 22a, 23a) au niveau de sa jonction avec le noyau central.

6. Implant (1) selon la revendication précédente, **caractérisée en ce que** lesdites trois pattes (21, 22, 23) présentent chacune une section transversale réduite (21 a, 22a, 23a) au niveau de sa jonction avec le noyau central.

7. Implant (1) selon l'une quelconque des revendications précédentes, **caractérisé en ce que** ladite deuxième partie (30) présente au moins deux jambes (32, 33) s'étendant selon l'axe longitudinal B à partir dudit noyau central.

8. Implant (1) selon la revendication précédente, **caractérisé en ce que** ladite deuxième partie présente trois jambes (31, 32, 33) s'étendant selon l'axe longitudinal B à partir dudit noyau central.

9. Implant (1) selon la revendication 7 ou 8, **caractérisé en ce que** la section en coupe dudit noyau central par un plan perpendiculaire audit axe longitudinal B présentant sensiblement ladite forme d'étoile à au moins trois branches (41, 42, 43), chaque dite jambe (31, 32, 33) s'étend sensiblement selon ledit axe longitudinal B à partir de l'extrémité libre d'une des branches dudit noyau central.

10. Implant (1) selon l'une quelconque des revendications 7 à 9, **caractérisé en ce que** lesdites jambes présentant globalement la forme d'un parallélépipède allongé, chaque jambe présente une section transversale réduite (31a, 32a, 33a) au niveau de sa jonction avec le noyau central.

11. Implant (1) selon l'une quelconque des revendications précédentes, **caractérisé en ce que** lesdits premiers moyens de fixation comprennent des crans d'accroche (24) situés sur lesdites pattes (21, 22, 23).

12. Implant (1) selon l'une quelconque des revendications 7 à 11, **caractérisé en ce que** lesdits deuxièmes moyens de fixation comprennent des crans d'accroche (34) situés sur lesdites jambes (31, 32, 33).

## Patentansprüche

1. Medizinisches Implantat (1), das dazu bestimmt ist, die Knochenfusion zwischen einem ersten Knochen (P2) und einem zweiten Knochen (P1) zu erlauben, wobei das Implantat einen ersten Teil (20) in allgemein länglicher Form umfasst und der eine Längsachse A aufweist, die dazu bestimmt ist, in den ersten Knochen eingeführt zu werden, und erste Befestigungsmittel (24) des Implantats in dem ersten Knochen umfasst, und einen zweiten Teil (30) mit ebenfalls insgesamt länglicher Form und der eine Längsachse B aufweist, die dazu bestimmt ist, in den zweiten Knochen eingeführt zu werden und zweite Befestigungsmittel (34) des Implantats in dem zweiten Knochen umfasst, wobei der erste und der zweite Teil (20, 30) miteinander durch einen zentralen Kern (40) verbunden sind, **dadurch gekennzeichnet, dass** der zentrale Knochen ein massiver Feststoff ist, dessen Querschnitt durch eine Ebene senkrecht zu der Längsachse A im Wesentlichen eine Sternform mit mindestens drei Schenkeln (41, 42, 43) aufweist, wobei der erste Teil mindestens drei Pratzen (21, 22, 23) aufweist, wobei sich jede Pratze im Wesentlichen entlang der Längsachse A ausgehend von dem freien Ende (41 a, 42a, 43a) eines Schenkels des zentralen Kerns erstreckt.

2. Implantat (1) nach Anspruch 1, **dadurch gekennzeichnet, dass** der Querschnitt des zentralen Kerns eine T-Form aufweist.

3. Implantat (1) nach Anspruch 1, **dadurch gekennzeichnet, dass** der Querschnitt des zentralen Kerns eine Y-Form aufweist.

4. Implantat (1) nach einem der vorhergehenden Ansprüche, **dadurch gekennzeichnet, dass** das Implantat einstückig ist.

5. Implantat (1) nach einem der vorhergehenden Ansprüche, **dadurch gekennzeichnet, dass** die Pratzen insgesamt die Form eines länglichen Parallelepipeds aufweisen, wobei mindestens eine Pratze einen verringerten Querschnitt (21 a, 22a, 23a) in dem Bereich ihrer Verbindung mit dem zentralen Kern aufweist.

6. Implantat (1) nach dem vorhergehenden Anspruch, **dadurch gekennzeichnet, dass** die drei Pratzen (21, 22, 23) jeweils einen verringerten Querschnitt (21 a, 22a, 23a) in dem Bereich ihrer Verbindung mit dem zentralen Kern aufweisen.

7. Implantat (1) nach einem der vorhergehenden Ansprüche, **dadurch gekennzeichnet, dass** der zweite Teil (30) mindestens zwei Beine (32, 33) aufweist, die sich entlang der Längsachse B ausgehend von dem zentralen Kern erstrecken.

8. Implantat (1) nach dem vorhergehenden Anspruch, **dadurch gekennzeichnet, dass** der zweite Teil drei Beine (31, 32, 33) aufweist, die sich entlang der Längsachse B ausgehend von dem zentralen Kern erstrecken.

9. Implantat (1) nach Anspruch 7 oder 8, **dadurch gekennzeichnet, dass** der Querschnitt des zentralen Kerns durch eine Ebene senkrecht zu der Längsachse B im Wesentlichen die Sternform mit mindestens drei Schenkeln (41, 42, 43) aufweist, wobei sich jedes Bein (31, 32, 33) im Wesentlichen entlang der Längsachse B ausgehend von dem freien Ende eines der Schenkel des zentralen Kerns erstreckt.

10. Implantat (1) nach einem der Ansprüche 7 bis 9, **dadurch gekennzeichnet, dass** die Beine insgesamt die Form eines länglichen Parallelepipeds aufweisen, wobei jedes Bein einen verringerten Querschnitt (31 a, 32a, 33a) in dem Bereich seiner Verbindung mit dem zentralen Kern aufweist.

11. Implantat (1) nach einem der vorhergehenden Ansprüche, **dadurch gekennzeichnet, dass** die ersten Befestigungsmittel Anhängrasten (24), die sich auf den Pratzen (21, 22, 23) befinden, umfassen.

12. Implantat (1) nach einem der Ansprüche 7 bis 11, **dadurch gekennzeichnet, dass** die zweiten Befestigungsmittel Anhängrasten (34), die sich auf den Beinen (31, 32, 33) befinden, umfassen.

## Claims

1. A medical implant (1) intended to allow bone fusion between a first bone (P2) and a second bone (P1), said implant comprising a first portion (20) of a generally elongated shape and presenting a longitudinal axis A, intended to be introduced into the first bone and comprising first fixation means (24) of said implant in said first bone, and a second portion (30), also of a generally elongated shape and presenting a longitudinal axis B, intended to be introduced into the second bone and comprising second fixation means (34) of said implant in said second bone, said first and second portions (20, 30) being connected together by a central core (40), **characterized in that** said central core is a solid body whose cross-section in a plane perpendicular to said longitudinal axis A presents substantially a star shape with at least three branches (41, 42, 43), said first portion having at least three tabs (21, 22, 23), each tab extending substantially along said longitudinal axis A from the free end (41 a, 42a, 43a) of one of the branches of said central core.

2. The implant (1) according to claim 1, **characterized in that** said cross-section of said central core is T-shaped

3. The implant (1) according to claim 1, **characterized in that** said cross-section of said central core is Y-shaped.

4. The implant (1) according to any one of the preceding claims, **characterized in that** said implant is in one piece.

5. The implant (1) according to any one of the preceding claims, **characterized in that** said tabs presenting generally the shape of an elongated parallelepiped, at least one tab presents a reduced cross-section (21 a, 22a, 23a) at its junction with the central core.

6. The implant (1) according to the preceding claim, **characterized in that** said three tabs (21, 22, 23) each have a reduced cross-section (21 a, 22a, 23a) at its junction with the central core.

7. The implant (1) according to any one of the preceding claims, **characterized in that** said second portion (30) presents at least two legs (32, 33) extending along the longitudinal axis B from said central core.

8. The implant (1) according to the preceding claim, **characterized in that** said second portion presents three legs (31, 32, 33) extending along the longitudinal axis B from said central core.

9. The implant (1) according to claim 7 or 8, **characterized in that** the cross-section of said central core in a plane perpendicular to said longitudinal axis B presenting substantially said star shape with at least three branches (41, 42, 43), each said leg (31, 32, 33) extends substantially along said longitudinal axis B from the free end of one of the branches of said central core.

10. The implant (1) according to any one of claims 7 to 9, **characterized in that** said legs presenting generally the shape of an elongated parallelepiped, each leg presents a reduced cross-section (31 a, 32a, 33a) at its junction with the central core.

11. The implant (1) according to any one of the preceding claims, **characterized in that** said first fixation means comprise hooking notches (24) located on said tabs (21, 22, 23).

12. The implant (1) according to any one of claims 7 to 11, **characterized in that** said second fixation means comprise hooking notches (34) located on said legs (31, 32, 33).
